# EUROPÄISCHE PATENTSCHRIFT

(11) **EP 0 816 318 B1**
(45) Veröffentlichungstag und Bekanntmachung des Hinweises auf die Patenterteilung: **13.06.2001**
(21) Anmeldenummer: 97109084.0
(22) Anmeldetag: 05.06.1997
(51) Int. Cl.: C07C 37/04, C07C 39/15

(54) **Verfahren zur Herstellung von 4-Hydroxydiphenyl**
Method for the preparation of 4-hydroxybiphenyl
Procédé pour la préparation de 4-hydroxybiphényle

(30) Priorität: 18.06.1996 DE 19624202
(43) Veröffentlichungstag der Anmeldung: 07.01.1998
(73) Patentinhaber: BAYER AG, 51368 Leverkusen (DE)
(72) Erfinder: Behre, Horst, Dr., 51519 Odenthal (DE); Fiege, Helmut, Dr., 51373 Leverkusen (DE); Rauchschwalbe, Günter, Dr., 51375 Leverkusen (DE)

(56) Entgegenhaltungen:
- US-A- 4 243 822
- US-A- 4 467 123
- CHEMICAL ABSTRACTS, vol. 111, no. 5, 31.Juli 1989 Columbus, Ohio, US; abstract no. 39001m, Seite 565; XP002052556 & JP 01 016 732 A (MITSUBISHI PETROCHEMICAL CO., LTD. ; SUGAI CHEMICAL INDUSTRY CO., LTD.)

## Beschreibung

Die vorliegende Erfindung betrifft ein Verfahren zur Herstellung von 4-Hydroxydiphenyl aus Diphenyl-4-sulfonsäure durch alkalische Hydrolyse und anschließende Temperung unter Druck.

4-Hydroxydiphenyl ist ein wichtiges Vorprodukt für die Herstellung von Pflanzenschutzmitteln und Emulgatoren.

Es ist aus der Patentliteratur bekannt, 4-Hydroxydiphenyl mit Hilfe einer Alkalischmelze aus Diphenyl-4-sulfonsäure herzustellen, wobei die Sulfo-Gruppe gegen die Hydroxy-Gruppe ausgetauscht wird. Zu dieser Alkalischmelze wird vielfach das Natriumsalz der genannten Sulfonsäure eingesetzt, das nach der Sulfierung von Diphenyl mit Schwefelsäure, Verdünnen des Sulfiergemisches mit Wasser, partieller Neutralisation mit Natronlauge, anschließender Filtration und Trockung gewonnen wird. In zahlreichen Patentschriften ist eine drucklose Alkalischmelze bei teilweise sehr hohen Temperaturen (mindestens 380°C) und langen Reaktionszeiten mit relativ schlechten Ausbeuten und Produktqualitäten beschrieben. Durch teilweisen Ersatz von NaOH durch das viel teurere KOH und durch die Verwendung von Zusätzen verschiedener Art wird versucht, die hohen Temperaturen und Viskositäten der Schmelzen zu mildern. (JP 71-30507; DE-OS 3 031 094; GB 2 071 090; US 2 368 361; JP 60-8720; JP 71-30508; JP 71-30509; JP 77-68154; JP 74-16417; JP 81-57728). Alle beschriebenen Verfahren können somit in Bezug auf die Reinheit und Ausbeute des erhaltenen Produktes oder in Bezug auf die anzuwendenden hohen Temperaturen oder die Umständlichkeit des Verfahrens, beispielsweise bei Anwendung von nachträglich abzutrennenden Zusätzen, nicht befriedigen.

In US 4 467 123 wird ein Verfahren zur Herstellung von 4-Hydroxydiphenyl aus Diphenyl-4-sulfonsäure durch alkalische Druckhydrolyse bei einer Temperatur zwischen 280 und 330°C und einem erhöhten Druck von bis zu 120 bar mit 3 bis 25 Mol wäßrigem Alkalihydroxid pro Äquivalent Sulfonat-Gruppe mit einer Konzentration von mindestens 50 Gew.-% beschrieben. Eine Nacharbeitung von Beispiel 2 der US 4 467 123 zur Herstellung von 4-Hydroxydiphenyl hat jedoch ergeben, daß dieses Produkt zwar in Bezug auf die organischen Nebenprodukte in hoher Reinheit, jedoch in Form eines thixotropen Feuchtproduktes mit einem sehr hohen Wasser-Gehalt von 65-70 Gew.-% anfällt, das praktisch nicht gehandhabt werden kann (siehe Vergleichsbeispiel).

Es wurde nun ein Verfahren zur Herstellung von 4-Hydroxydiphenyl in Form eines nicht thixotropen Feuchtproduktes aus Diphenyl-4-sulfonsäure durch alkalische Hydrolyse mit Alkalihydroxid gefunden, das dadurch gekennzeichnet ist, daß man das alkalische Reaktionsgemisch nach Verdünnen mit Wasser und Neutralisation mit einer Mineralsäure unter erhöhtem Druck bei einer Temperatur >115°C und einem pH-Wert von 0 bis <6,5 tempert.

Bei dem erfindungsgemäßen Verfahren kann das 4-Hydroxydiphenyl auf jede beliebige Weise durch alkalische Hydrolyse von Diphenyl-4-sulfonsäure mit Alkalihydroxid hergestellt werden, beispielsweise durch eine drucklose Backschmelze (Pulverschmelze), gegebenenfalls auch in Gegenwart eines sehr hoch siedenden inerten organischen Mediums, oder durch eine alkalische Druckhydrolyse.

Bevorzugt wird das 4-Hydroxydiphenyl nach dem erfindungsgemäßen Verfahren durch eine alkalische Druckhydrolyse bei einer Temperatur von 290-340°C, insbesondere bei 310 bis 330°C, und bei einem Druck von bis zu 120 bar, beispielsweise 2 bis 120 bar, bevorzugt bei 5 bis 80 bar, besonders bevorzugt bei 10 bis 40 bar durchgeführt. Diese Drücke können der Eigendruck der Reaktionsmischung sein oder zusätzlich zum Eigendruck durch Aufpressen von Inertgas, beispielsweise Stickstoff, erhöht werden. Der Eigendruck des Reaktionsgemisches ist in einer dem Fachmann im Prinzip geläufigen Weise von den Mischungs- und Konzentrationsverhältnissen im Reaktionsgemisch und der eingestellten Reaktionstemperatur abhängig. Diese Abhängigkeit gilt besonders für den Anteil Wasser im Reaktionsgemisch. Für den Fall, daß ein Druck im unteren Teil der genannten Bereiche eingestellt werden soll, kann dies daher durch Ablassen von Wasserdampf aus dem Reaktionsgemisch über ein Druckhalteventil erfolgen. Als wäßriges Alkalihydroxid für das erfindungsgemäße Verfahren seien beispielsweise wäßrige Lösungen von Natriumhydroxid oder Kaliumhydroxid, bevorzugt Natriumhydroxid, mit einer Konzentration von mindestens 50 Gew.-% Alkalihydroxid, bezogen auf das Gesamtgewicht der wäßrigen Lösung, genannt. Beispielsweise sei eine Konzentration von 50 bis 96 Gew.-%, bevorzugt von 60 bis 95 Gew.-%, besonders bevorzugt von 65 bis 85 Gew.-% genannt. Die Menge des wäßrigen Alkalihydroxids wird hierbei so bemessen, daß 3 bis 25 Mol Alkalihydroxid pro 1 Mol Alkali-diphenylsulfonat, bevorzugt 5 bis 12 Mol Alkalihydroxid pro 1 Mol Alkali-diphenylsulfonat im Reaktionsgemisch vorhanden sind. Die genannten molaren Mengen Alkalihydroxid beziehen sich hierbei auf die Sulfonat-Gruppe, die beispielsweise das Natrium- oder das Kaliumsalz der Diphenyl-4-sulfonsäure ist.

Die Herstellung der Diphenyl-4-sulfonsäure geschieht in bekannter Weise durch Sulfonieren von Diphenyl mit Schwefelsäure. Die hierbei erhältlichen Sulfonsäuren werden sodann in bekannter Weise zu den entsprechenden Sulfonaten neutralisiert. Diese Neutralisation kann außerhalb des Reaktionsgemisches für das erfindungsgemäße Verfahren erfolgen, so daß in das erfindungsgemäße Verfahren ein bereits neutralisiertes Sulfonat eingesetzt wird. Die Neutralistion zum Sulfonat kann jedoch auch im erfindungsgemäßen Verfahren erfolgen, so daß man die freie Diphenyl-4-sulfonsäure in das erfindungsgemäße Verfahren einsetzt. Erfindungsgemäß ist es weiterhin möglich, die Diphenyl-4-sulfonsäure oder ihre Alkalisalze in trockener oder wasserfeuchter Form oder in Form ihrer wäßrigen Lösung einzusetzen. Erfindungsgemäß können entweder die reine Diphenyl-4-sulfonsäure oder Sulfiergemische eingesetzt werden, die Diphenyl-4-sulfonsäure neben nicht umgesetzter Schwefelsäure und neben weiteren Diphenylmono- und -disulfonsäuren enthält. Bevorzugt wird für das erfindungsgemäße Verfahren die Diphenyl-4-sulfonsäure in Form des wasserfeuchten Natriumsalzes eingesetzt. Da zur Herstellung des 4-Hydroxydiphenyls die molare Menge wäßriges Alkalihydroxid auf die Sulfonatgruppe bezogen ist, muß beim Einsatz der freien Diphenyl-4-sulfonsäure bzw. beim Einsatz von Diphenyl-4-sulfonsäure enthaltenden Sulfiergemischen eine zusätzliche Menge Alkalihydroxid zugefügt werden, die zur vollständigen Neutralisation aller sauren Gruppen ausreicht.

Bei dem erfindungsgemäßen Verfahren wird das alkalische Reaktionsgemisch mit Wasser verdünnt und mit einer Mineralsäure bis zu einem pH-Wert von 0 bis <6,5, vorzugsweise 3 bis <6,5, insbesondere 5 bis <6,5 neutralisiert bzw. schwach angesäuert, um das 4-Hydroxydiphenyl aus dem bei der Alkalischmelze zunächst gebildeten Alkalisalz in Freiheit zu setzen. Als Mineralsäuren im erfindungsgemäßen Verfahren können beispielsweise Salzsäure und/oder Schwefelsäure eingesetzt werden. Bevorzugt wird Schwefelsäure, beispielsweise mit einem Gehalt von 10 bis 100 Gew.-%, eingesetzt. Die zur Verdünnung des alkalischen Reaktionsgemisches notwendige Wassermenge wird bei dem erfindungsgemäßen Verfahren so gewählt, daß die bei der Neutralisation/Ansäuerung gebildeten Alkalisalze, beispielsweise Natriumsulfat und/oder Natriumchlorid, bei der Isolierung des nach dem erfindungsgemäßen Verfahren hergestellten 4-Hydroxydiphenyls gelöst bleiben oder gelöst werden.

Wesentlich für die Isolierung von 4-Hydroxydiphenyl in Form eines nicht thixotropen Feuchtproduktes nach dem erfindungsgemäßen Verfahren ist, daß das neutralisierte/angesäuerte Reaktionsgemisch unter erhöhtem Druck bei einer Temperatur >115°C und einem pH-Wert von <6,5 getempert wird. Bevorzugt wird diese Temperung bei Temperaturen von 120 bis 170°C, besonders bevorzugt bei 125 bis 155°C, insbesondere bei 130 bis 145°C, durchgeführt.

Die Temperungszeit ist abhängig von der gewählten Temperatur und beträgt im allgemeinen 5 min bis 5 h und im besonders bevorzugten Temperaturbereich von 130 bis 145°C ungefähr 15 min bis 3 h.

Im einzelnen wird das erfindungsgemäße Verfahren in der bevorzugten Ausführungsform wie folgt durchgeführt:

Diphenyl-4-sulfonsäure-Na-Salz wird mit etwa 70 gew.-%iger NaOH zu 4-Oxydiphenyl-Na-Salz umgesetzt (etwa 320°C; 16-26 bar), wobei das Molverhältnis NaOH zu Diphenyl-4-sulfonat etwa 10:1 betragen soll. Das Reaktionsgemisch wird durch Direkt-Entspannung bei 320°C auf eine Wasser-Vorlage oder durch Einpumpen von Wasser in das Reaktionsgemisch nach vorheriger Kühlung auf 200°C auf einen Gehalt von etwa 12 Gew.-% 4-Hydroxydiphenyl verdünnt. Die resultierende alkalische 4-Hydroxydiphenyl-Na-Salz-Suspension wird simultan mit etwa 50 gew.-%iger H₂SO₄ bei 90°C und einem pH-Wert von 5,5 in eine Wasservorlage eindosiert. Die anfallende wäßrige, Na₂SO₃/NaHSO₃-haltige 4-Hydroxydiphenyl-Suspension mit einem Gehalt von etwa 4,3 Gew.-% 4-Hydroxydiphenyl wird anschließend bei 130-135°C und etwa 2 bar und während etwa 2 h getempert, mit 10 K/h auf 100°C und anschließend weiter auf 60°C kaltgerührt. Das sehr gut filtrierende, grob kristalline Produkt wird bei 60°C über eine Glassinternutsche isoliert und mit Wasser gewaschen. Man erhält ein fast farbloses, rieselfähiges, nicht thixotropes, weitgehend Na₂SO₄-freies Produkt mit einem drastisch verringerten Wasser-Gehalt von 10-20 Gew.-% gegenüber dem ohne erfindungsgemäße Temperung erhaltenen 65-70 Gew.-% H₂O enthaltenden, stark thixotropen Produkt.

Das erfindungsgemäße Verfahren kann diskontinuierlich und kontinuierlich durchgeführt werden.

### Beispiel 1

Ein 5 1-Nickel-Autoklav wurde nacheinander mit 2000 g NaOH (70 %) und 953 g Diphenyl-4-sulfonsäure-Na-Salz (87,2 Gew.-% freie Säure, MG 234) beschickt. Nach Spülen mit Stickstoff wurde die Reaktionsmischung (Suspension) im Verlaufe von ca. 2 h auf 320°C erhitzt, wobei der Rührer erst ab 180°C eingeschaltet wurde, und ca. 7 h bei 320°C und einem Druck von 16-26 bar nachgerührt. Zur Verdünnung der Reaktionsmischung wurden nach Kaltrühren auf 200°C (ca. 2 h) 2000 g Wasser eingepumpt und der Autoklav nach weiterem Abkühlen auf ca. 75°C vollständig entleert. Die erhaltene alkalische 4-Hydroxydiphenyl-Na-Salz-Suspension mit einem Gehalt von ca. 12 Gew.-% 4-Hydroxydiphenyl (aus HPLC), MG 170 wurde simultan mit 3370 g H₂SO₄ (50 %) bei 90°C und einem pH-Wert von 5,5 im Verlaufe von ca. 1 h in eine Vorlage von 5000 g Wasser eindosiert, wobei ca. 13320 g wäßrige, Na₂SO₃/NaHSO₃-haltige 4-Hydroxydiphenyl-Suspension mit einem Gehalt von ca. 4,3 Gew.-% 4-Hydroxydiphenyl (aus HPLC) erhalten wurden. In einem 1,6 1-Glas-Autoklaven wurden 900 g dieser wäßrigen 4-Hydroxydiphenyl-Suspension vorgelegt. Die Reaktionsmischung wurde im Verlaufe von ca. 1/2 h unter gutem Rühren (750 U/min) auf 130-135°C erhitzt, wobei sich ein Druck von ca. 2 bar einstellte und ein leichtes Sintern von 4-Hydroxydiphenyl zu beobachten war, 2 h bei 130-135°C getempert, mit 10 K/h auf 100°C und anschließend weiter auf 60°C kaltgerührt. Das sehr gut filtrierende, grob kristalline Produkt wurde bei 60°C über eine Glassinternutsche isoliert und mit insgesamt 100 g Wasser in zwei gleichen Portionen gewaschen. Es wurden 46 g 4-Hydroxydiphenyl, feucht (fast farbloses, rieselfähiges, nicht thixotropes Produkt) erhalten.

Der durch HPLC bestimmte Gehalt des isolierten Produktes betrug:

| | |
|---|---|
| 83,0 Gew.-% | 4-Hydroxydiphenyl, MG 170 |
| 17,0 Gew.-% | Wasser |
| <0,2 Gew.-% | Sulfat, MG 96. |

Die Ausbeute an 4-Hydroxydiphenyl betrug 94 % der theoretischen Ausbeute, bezogen auf eingesetzte Diphenyl-4-sulfonsäure.

### Beispiel 2 (zum Vergleich)

Eine Menge von 900 g einer wie in Beispiel 1 hergestellten wäßrigen Na₂SO₃/NaHSO₃-haltigen 4-Hydroxydiphenyl-Suspension mit einem Gehalt von ca. 4,3 Gew.-% 4-Hydroxydiphenyl (aus HPLC) wurde von 90°C auf 60°C kaltgerührt. Das gut filtrierende Produkt wurde bei 60°C über eine Glassinternutsche isoliert und mit insgesamt 300 g Wasser in zwei gleichen Portionen gewaschen. Es wurden 127 g 4-Hydroxydiphenyl, feucht (fast farbloses, stark thixotropes Produkt) erhalten.

Der durch HPLC bestimmte Gehalt des isolierten Produktes betrug:

| | |
|---|---|
| 31,0 Gew.-% | 4-Hydroxydiphenyl, MG 170 |
| 69,0 Gew.-% | Wasser |
| <0,2 Gew.-% | Sulfat, MG 96 |

Die Ausbeute an 4-Hydroxydiphenyl betrug 94 % der theoretischen Ausbeute, bezogen auf eingesetzte Diphenyl-4-sulfonsäure. Die Handhabung und Weiterverarbeitung des stark thixotropen Produktes war äußerst problematisch (z.B. Lagerung, Befüllen und Entleeren von Gebinden, schwieriger Transfer zu einem geeigneten Trockungsaggregat, unwirtschaftlich hohe Trocknungskosten wegen des hohen H₂O-Gehalts).

## Patentansprüche

1. Verfahren zur Herstellung von 4-Hydroxydiphenyl in Form eines nicht thixotropen Feuchtproduktes aus Diphenyl-4-sulfonsäure durch alkalische Hydrolyse mit Alkalihydroxid, dadurch gekennzeichnet, daß man das alkalische Reaktionsgemisch nach Verdünnen mit Wasser und Neutralisation mit einer Mineralsäure unter erhöhtem Druck bei einer Temperatur oberhalb von 115°C und einem pH-Wert von 0 bis unterhalb 6,5 tempert.

2. Verfahren gemäß Anspruch 1, dadurch gekennzeichnet, daß die Temperung des neutralisierten Reaktionsgemisches unter erhöhtem Druck bei einem pH-Wert von <6,5 bei Temperaturen von 120 bis 170°C, besonders bevorzugt bei 125 bis 155°C, insbesondere bei 130 bis 145°C, durchgeführt wird.

3. Verfahren gemäß Anspruch 1 und 2, dadurch gekennzeichnet, daß das 4-Hydroxydiphenyl durch eine alkalische Druckhydrolyse aus Diphenyl-4-sulfonsäure bei einer Temperatur von 290-340°C, insbesondere bei 310 bis 330°C, bei einem Druck von 2 bis 120 bar, bevorzugt bei 5 bis 80 bar, besonders bevorzugt bei 10 bis 40 bar und bei einer Alkalihydroxid-Konzentration von 50 bis 96 Gew.-%, bevorzugt von 60 bis 95 Gew.-%, besonders bevorzugt von 65 bis 85 Gew.-%, erhalten wird, wobei die Menge des wäßrigen Alkalihydroxids so bemessen wird, daß 3 bis 25 Mol Alkalihydroxid pro 1 Mol Alkali-diphenylsulfonat, bevorzugt 5 bis 12 Mol Alkalihydroxid pro 1 Mol Alkali-diphenylsulfonat, im Reaktionsgemisch vorhanden sind.

## Claims

1. Process for the preparation of 4-hydroxybiphenyl in the form of a nonthixotropic moist product from biphenyl-4-sulphonic acid by alkaline hydrolysis with alkali metal hydroxide, characterized in that the alkaline reaction mixture, after dilution with water and neutralization with a mineral acid, is heated at elevated pressure at a temperature above 115°C and at a pH of 0 to below 6.5.

2. Process according to Claim 1, characterized in that the neutralized reaction mixture is heated at elevated pressure at a pH of < 6.5 at temperatures of 120 to 170°C, particularly preferably 125 to 155°C, in particular at 130 to 145°C.

3. Process according to Claims 1 and 2, characterized in that the 4-hydroxybiphenyl is obtained by an alkaline pressurized hydrolysis of biphenyl-4-sulphonic acid at a temperature of 290-340°C, in particular at 310 to 330°C, at a pressure of 2 to 120 bar, preferably at 5 to 80 bar, particularly preferably at 10 to 40 bar, and at an alkali metal hydroxide concentration of 50 to 96% by weight, preferably 60 to 95% by weight, particularly preferably 65 to 85% by weight, with the amount of the aqueous alkali metal hydroxide being such that 3 to 25 mol of alkali metal hydroxide are present in the reaction mixture per 1 mol of alkali metal biphenylsulphonate, preferably 5 to 12 mol of alkali metal hydroxide per 1 mol of alkali metal biphenylsulphonate.

## Revendications

1. Procédé de préparation de 4-hydroxydiphényle sous forme d'un produit humide non thixotrope à partir d'acide diphényl-4-sulfonique par hydrolyse basique avec un hydroxyde de métal alcalin, caractérisé en ce que, après l'avoir dilué avec de l'eau et neutralisé avec un acide inorganique, on réchauffe le mélange réactionnel basique à une température supérieure à 115°C sous pression élevée, à un pH allant de 0 à une valeur inférieure à 6,5.

2. Procédé selon la revendication 1, caractérisé en ce que le réchauffage du mélange réactionnel neutralisé s'effectue sous haute pression, à un pH inférieur à 6,5, à des températures de 120 à 170°C, de façon particulièrement préférée de 125 à 155°C, en particulier de 130 à 145°C.

3. Procédé selon les revendications 1 et 2, caractérisé en ce que l'on obtient le 4-hydroxydiphényle à partir de d'acide diphényl-4-sulfonique par hydrolyse basique sous pression à une température de 290-340°C, en particulier de 310 à 330°C, sous une pression de 2 à 120 bars, de préférence de 5 à 80 bars, de façon particulièrement préférée de 10 à 40 bars, et à une concentration d'hydroxyde de métal alcalin de 50 à 96 % en masse, de préférence de 60 à 95 % en masse, de façon particulièrement préférée de 65 à 85 % en masse, en déterminant la quantité d'hydroxyde de métal alcalin aqueux de façon que le mélange réactionnel contienne 3 à 25 mol d'hydroxyde de métal alcalin par mol de diphénylsulfonate de métal alcalin, de préférence 5 à 12 mol d'hydroxyde de métal alcalin par mol de diphénylsulfonate de métal alcalin.
